(19) Europäisches Patentamt
European Patent Office
Office européen des brevets

(11) Numéro de publication : **0 296 978 B1**

# (12) FASCICULE DE BREVET EUROPEEN

(45) Date de publication du fascicule du brevet : **11.09.91 Bulletin 91/37**

(51) Int. Cl.$^5$ : **C07D 207/273**, A61K 31/40

(21) Numéro de dépôt : **88401586.8**

(22) Date de dépôt : **23.06.88**

(54) **Nouveaux dérivés de 1-benzyl 2-oxo 5-alcoxy pyrrolidine, leur procédé de préparation, leur application comme médicaments et les compositions les renfermant.**

(30) Priorité : **25.06.87 IT 2104687**

(43) Date de publication de la demande : **28.12.88 Bulletin 88/52**

(45) Mention de la délivrance du brevet : **11.09.91 Bulletin 91/37**

(84) Etats contractants désignés : **AT BE CH DE ES FR GB GR IT LI LU NL SE**

(56) Documents cités :
**FR-A- 2 380 257**
**CHEMICAL ABSTRACTS, vol. 100, 1984, page 565, résumé no. 51412b, Columbus, Ohio, US; W.N. SPECKAMP et al.: "Carbo]-carbon bond forming reactions of omega-hydroxy lactams in acid and neutral medium"**

(73) Titulaire : **ROUSSEL-UCLAF**
**35, boulevard des Invalides**
**F-75007 Paris (FR)**

(72) Inventeur : **Toja, Emilio**
**Via Piezzo 80**
**Milano (IT)**
Inventeur : **Gorini, Carlo**
**Via Orcagna 4**
**Milano (IT)**
Inventeur : **Barzaghi, Fernando**
**Via Monteverdi 21**
**I-20052 Monza (MI) (IT)**
Inventeur : **Galliani, Giulio**
**13, Via Silva**
**I-1 Monza (MI) (IT)**

(74) Mandataire : **Tonnellier, Marie-José et al**
**111, route de Noisy B.P. no 9**
**F-93230 Romainville (FR)**

EP 0 296 978 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

L'invention concerne de nouveaux dérivés de la 1-benzyl 2-oxo 5-alcoxy pyrrolidine, leur procédé de préparation, leur application comme médicaments et les compositions pharmaceutiques les renfermant.

L'invention a pour objet les composés de formule (I) :

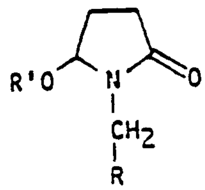

(I)

dans laquelle R' représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b]-pyranyle, benzoxazolyle ou morpholinyle, R étant éventuellement substitué par un ou plusieurs substituants choisis dans le groupe constitué par le radical hydroxy, le radical acétoxy, le radical méthoxy ou le radical benzyloxy, le radical méthyle, éthyle, propyle ou isopropyle, le radical éthényle ou le radical éthynyle, le fluor, le chlore ou le brome, les groupements $CF_3$, $SCF_3$, $OCF_3$, $NO_2$, $NH_2$ ou C≡N, le radical phényle, les groupements acyles ou alcoxycarbonyles renfermant de 2 à 8 atomes de carbone et les groupements alcoylsulfonyles renfermant de 1 à 6 atomes de carbone, à la condition que si R est un radical phényle non substitué, R' ne représente ni un atome d'hydrogène, ni un radical méthyle, ni un radical éthyle.

Par radical alcoyle, on entend de préférence un radical renfermant de 3 à 10 atomes de carbone, par exemple, le radical n-propyle, isopropyle, n-butyle, isobutyle, terbutyle, n-pentyle, n-hexyle, n-heptyle, n-octyle, cyclopropyle, cyclobutyle, cyclopentyle ou cyclohexyle.

Par radical alcényle, on entend de préférence un radical éthényle, propényle, butényle.

Par radical acyle, on entend de préférence un radical acétyle, propionyle ou butyryle.

L'invention a plus particulièrement pour objet les composés de formule I dans lesquels R représente un radical phényle éventuellement substitué ainsi que ceux dans lesquels R' représente un radical alcoyle linéaire, ramifié ou cyclique, renfermant de 3 à 12 atomes de carbone comme par exemple, le radical n-pentyle, n-hexyle, n-heptyle ou n-octyle.

Parmi les composés préférés de l'invention, on peut citer les composés des exemples 6 et 7.

Les composés de formule I dans lesquels R représente un radical phényle non substitué et R' un atome d'hydrogène ou un radical méhyle ou éthyle sont des produits chimiques connus décrits par exemple dans Chem. Abstr. (1984), 100 : 51412 b, Chem. Abstr. (1973) 79 5204 K, J.C.S. Chem. com. 134 (1982) ; J. Org. Chem. 49 1149 (1984), toutefois, la littérature ne mentionne aucune propriété pharmacologique pour ces composés. On vient de découvrir que les composés répondant à la formule générale I ci-dessus présentent d'intéressantes propriétés pharmacologiques : ils retardent l'extinction de la réponse d'évitement conditionné, ils retardent la disparition de la réponse apprise. Ils favorisent l'attention, la vigilance et la mémorisation.

L'invention a donc pour objet les produits de formule (I) en tant que médicaments, utiles notamment dans le traitement des asthénies intellectuelles ou nerveuses, des défaillances de la mémoire, de la sénescence, du surmenage intellectuel.

L'invention a plus particulièrement pour objet en tant que médicament, les produits des exemples 6 et 7.

La posologie usuelle est variable selon l'affection en cause, le sujet traité et la voie d'administration, elle peut être comprise entre 50 mg et 3000 mg/jour, par exemple entre 150 et 1500 mg/jour en une ou plusieurs prises pour le produit de l'exemple 1 administré par voie orale.

La présente invention a également pour objet les compositions pharmaceutiques renfermant comme principe actif au moins un produit de formule (I).

Les compositions pharmaceutiques de l'invention peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés

paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) :

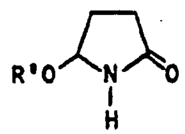

(II)

dans laquelle R' conserve la même signification que précédemment, à l'action d'un composé de formule (III):

$RCH_2$-Hal (III)

dans laquelle R conserve la même signification que précédemment et Hal représente un atome d'halogène, en présence d'une base pour obtenir le composé de formule (I) correspondant.

Dans un mode de réalisation préféré du procédé de l'invention :

— Hal représente un atome de brome,

— la base forte utilisée est un hydroxyde alcalin comme la soude ou la potasse et l'on opère en présence d'un halogénure de tétrabutylammonium, comme par exemple, le bromure de tétrabutylammonium,

— la condensation des composés (II) et (III) a lieu au sein d'un solvant choisi dans le groupe constitué par le tétrahydrofuranne, le benzène, le diméthylformamide, le diméthylsulfoxyde ou l'éther diéthylique du diéthylène glycol.

Les produits de formule (II) utilisés comme produits de départ sont des produits connus d'une façon générale, qui peuvent être préparés selon le procédé décrit dans Tetrahedron 31, 1437 (1975) ou Tetrahedron 41, 2007 (1985) ou selon le procédé décrit dans Heterocycles 22, 1733 (1984). Certaines préparations de produits de formule (II) sont données ci-après dans la partie expérimentale.

Les exemples suivants illustrent l'invention sans toutefois la limiter.

**Exemple 1 : 1-benzyl 2-oxo 5-n-propyloxy pyrrolidine.**

A un mélange de 4 g de 5-(n-propyloxy) pyrrolidine 2-one, 0,4 g de bromure de n-tétrabutylammonium et 2,34 g de potasse hydratée à 85% dans 70 cm3 de tétrahydrofuranne, on ajoute une solution de 4,78 g de bromure de benzyle dans 25 cm3 de tétrahydrofuranne en opérant entre 20°C et 30°C. On agite pendant 1 heure, filtre puis évapore sous pression réduite. Le résidu est chromatographié sur silice (éluant : acétate d'éthyle-n-hexane 1-1). On obtient 4 g de produit attendu.

| Analyse : $C_{14}H_{19}NO_2$ | | | |
|---|---|---|---|
| Calculé : | C% 72,07 | H% 8,21 | N% 6,00 |
| Trouvé : | 72,20 | 8,16 | 6,22 |

**Exemple 2 : 1-benzyl 2-oxo 5-isopropoxy pyrrolidine.**

A une suspension de 2,5 g de 5-isopropoxy pyrrolidin 2-one, 1,23 g de potasse hydratée à 85%, 0,25 g de bromure de tétra n-butylammonium dans 50 cm3 de tétrahydrofuranne, on ajoute une solution de 2,99 g de bromure de benzyle sans dépasser 30°C. On agite pendant 1 heure à température ambiante. On filtre l'insoluble, évapore le solvant. Le résidu (4 g) est distillé à 200°C sous 0,05 mbar. On chromatographie le distillat sur silice (éluant : acétate d'éthyle-n-hexane 1-1). On obtient 2,9 g de produit attendu.

| Analyse : $C_{14}H_{19}NO_2$ | | | |
|---|---|---|---|
| Calculé : | C% 72,07 | H% 8,21 | N% 6,00 |
| Trouvé : | 71,81 | 8,06 | 6,05 |

**Exemple 3 : 1-benzyl 2-oxo 5-n-butyloxy pyrrolidine.**

A une suspension de 4 g de 5-n-butyloxy pyrrolidine 2-one, 1,79 g de potasse hydratée à 85% et 0,4 g de bromure de n-tétrabutylammonium dans 80 cm3 de tétrahydrofuranne, on ajoute une solution de 4,35 g de bromure de benzyle sans dépasser 30°C. On agite pendant 1 heure à température ambiante. On filtre l'insoluble, évapore le solvant. On distille le résidu (4,7 g) à 230°C sous 0,05 mbar puis chromatographie le distillat sur silice (éluant : acétate d'éthyle-n-hexane 1-1). On obtient 3,5 g de produit attendu.

| Analyse : | $C_{15}H_{21}NO_2$ | | |
|---|---|---|---|
| Calculé : | C% 72,84 | H% 8,56 | N% 5,66 |
| Trouvé : | 72,66 | 8,65 | 5,79 |

**Exemple 4 : 1-benzyl 2-oxo 5-n-pentyloxy pyrrolidine.**

A un mélange de 4 g de 5-(n-pentyloxy) pyrrolidin 2-one, 0,4 g de bromure de n-tétrabutylammonium et 1,96 g de potasse hydratée à 85% dans 60 cm3 de tétrahydrofuranne, on ajoute sans dépasser 30°C une solution de 3,99 g de bromure de benzyle dans 20 cm3 de tétrahydrofuranne. On agite 1 heure à température ambiante, filtre, évapore à secet chromatographie le résidu sur silice (éluant : acétate d'éthyle-n-hexane 1-1). On obtient 4,5 g de produit attendu.

| Analyse : | $C_{16}H_{23}NO_2$ | | |
|---|---|---|---|
| Calculé : | C% 73,53 | H% 8,87 | N% 5,36 |
| Trouvé : | 73,71 | 8,94 | 5,48 |

**Exemple 5 : 1-benzyl 2-oxo 5-n-hexyloxy pyrrolidine.**

A un mélange de 3,5 g de 5-(n-hexyloxy) pyrrolidin 2-one, 1,58 g de potasse hydratée à 85% et 0,35 g de bromure de tétra-n-butylammonium dans 50 cm3 de tétrahydrofuranne, on ajoute à 20°C-25°C une solution de 3,23 g de bromure de benzyle dans 20 cm3 de tétrahydrofuranne. On agite pendant 1 heure à température ambiante, filtre, évapore à sec sous pression réduite et chromatographie le résidu sur silice (éluant : acétate d'éthyle-n-hexane 1-2). on Obtient 3,64 g de produit attendu.

| Analyse : | $C_{17}H_{25}NO_2$ | | |
|---|---|---|---|
| Calculé : | C% 74,14 | H% 9,15 | N% 5,09 |
| Trouvé : | 74,44 | 9,25 | 5,09 |

**Exemple 6 : 1-benzyl 2-oxo 5-n-heptyloxy pyrrolidine.**

A un mélange de 4 g de 5-(n-heptyloxy) pyrrolidin 2-one, 1,68 g de potasse hydratée à 85% et 0,4 g de bromure de tétra-n-butylammonium dans 55 cm3 de tétrahydrofuranne, on ajoute une solution de 3,43 g de bromure de benzyle dans 20 cm3 de tétrahydrofuranne en opérant à 20°C-25°C. On agite pendant 1 heure à température ambiante, filtre, évapore le solvant sous pression réduite. On chromatographie le résidu sur silice (éluant : acétate d'éthyle-n-hexane 1-2) et obtient 4,54 g de produit attendu.

| Analyse : | $C_{18}H_{27}NO_2$ | | |
|---|---|---|---|
| Calculé : | C% 74,7 | H% 9,40 | N% 4,84 |
| Trouvé : | 74,54 | 9,36 | 4,93 |

**Exemple 7 : 1-benzyl 2-oxo 5-n-octyloxy pyrrolidine.**

A un mélange de 2 g 5-n-octyloxy pyrrolidin 2-one, 0,65 g de potasse à 85% et 0,2 g de bromure de tétra-n-butylammonium dans 40 cm3 de tétrahyorofuranne, on ajoute une solution de 1,6 g de bromure de benzyle dans 5 cm3 de tétrahydrofuranne sans dépasser 30°C. On agite pendant 2 heures à température ambiante, filtre, évapore à sec et chromatographie le résidu sur alumine (éluant : acétate d'éthyle-cyclohexane 1-1). On obtient 2 g du produit recherché.

| Analyse : | $C_{19}H_{29}NO_2$ | | |
|---|---|---|---|
| Calculé : | C% 75,20 | H% 9,63 | N% 4,62 |
| Trouvé : | 74,98 | 9,44 | 4,49 |

**Exemple 8 : 1-(4-méthoxybenzyl) 2-oxo 5-éthoxy pyrrolidine.**

A un mélange de 2,25 g de 5-éthoxy pyrrolidin 2-one, 1,23 g de potasse hydratée et 0,2 g de bromure de tétra-n-butylammonium dans 45 cm3 de tétrahydrofuranne, on ajoute sans dépasser 30°C une solution de 3,5 g de bromure de 4-méthoxybenzyle dans 14 cm3 de tétrahydrofuranne. On agite pendant 1 heure à température ambiante, filtre, évapore le solvant sous pression réduite, reprend le résidu à l'eau, extrait à l'acétate d'éthyle, sèche et évapore le solvant sous pression réduite. Le résidu est distillé à 245°C sous 0,2 mbar. On obtient 3,10 g de produit attendu.

| Analyse : | $C_{14}H_{19}NO_3$ | | |
|---|---|---|---|
| Calculé : | C% 67,45 | H% 7,68 | N% 5,62 |
| Trouvé : | 67,24 | 7,59 | 5,66 |

**Exemple 9 : 1-benzyl 2-oxo 5-n-nonyloxy pyrrolidine.**

A un mélange de 6 g de 5-n-nonyloxy pyrrolidin 2-one, 1,83 g de potasse à 85% et 0,6 g de bromure de tétrabutylammonium dans 120 cm3 de tétrahydrofuranne, on ajoute sans dépasser 30°C 4,51 g de bromure de benzyle dans 9 cm3 de tétrahydrofuranne. On agite pendant 2 heures, filtre, puis évapore à sec. On chromatographie le résidu en éluant avec un mélange acétate d'éthyle-n-hexane (1-1) et obtient 7 g de produit attendu.

| Analyse : | $C_{20}H_{31}NO_2$ | | |
|---|---|---|---|
| Calculé : | C% 75,66 | H% 9,84 | N% 4,41 |
| Trouvé : | 75,55 | 9,87 | 4,35 |

**Exemple 10 : 1-benzyl 2-oxo 5-n-décyloxy pyrrolidine.**

A un mélange de 3 g de 5-n-décyloxy pyrrolidin-2-one, 0,86 g de potasse à 85% et 0,3 g de bromure de tétrabutylammonium dans 60 cm3 de tétrahydrofuranne, on ajoute sans dépasser 30°C une solution de 2,12 g de bromure de benzyle dans 5 cm3 de tétrahydrofuranne. On agite pendant 2 heures à température ambiante, on filtre puis évapore à sec. On chromatographie le résidu sur silice (éluant : acétate d'éthyle-n-héxane 1-1) et obtient 3 g de produit attendu.

| Analyse : | $C_{21}H_{33}NO_2$ | | |
|---|---|---|---|
| Calculé : | C% 76,09 | H% 10,03 | N% 4,23 |
| Trouvé : | 75,88 | 10,01 | 4,17 |

**Exemple 11 : 1-(3-fluoro) benzyl 2-oxo 5-n-octyloxy prrrolidine.**

A un mélange de 2,26 g de 5-n-octyloxy pyrrolidin-2-one, 0,23 g de bromure de tétrabutylammonium et 0,65 g de potasse dans 40 cm3 de tétrahydrofuranne, on ajoute sans dépasser 30°C une solution de 2 g de bromure de 3-fluorobenzyle dans 20 cm3 de tétrahydrofuranne. On agite 3 heures à température ambiante, filtre et évapore le solvant sous pression réduite. On chromatographie le résidu sur silice (éluant : acétate d'éthyle-n-hexane 1-1) et obtient 2,25 g de produit attendu.

| Analyse : | $C_{19}H_{28}FNO_2$ | | |
|---|---|---|---|
| Calculé : | C% 70,99 | H% 8,78 | N% 4,36 |
| Trouvé : | 70,68 | 8,61 | 4,47 |

**Exemple 12 : 1-(4-fluoro) benzyl 2-oxo 5-n-octyloxy pyrrolidine.**

A un mélange de 2 g de 5-n-octyloxy pyrrolidin 2-one, 0,2 g de bromure de tétrabutylammonium et 0,58 g de potasse dans 40 cm3 de tétrahyorofuranne sans dépasser 30°C, on ajoute une solution de 1,77 g de bromure de 4-fluorobenzyle dans 20 cm3 de tétrahydrofuranne. On agite 3 heures à température ambiante, filtre et évapore le solvant sous pression réduite. On chromatographie le résidu sur silice (éluant : acétate d'éthyle-n-hexane 1-1) et obtient 2,6 g de produit attendu.

| Analyse : | $C_{19}H_{28}FNO_2$ | | |
|---|---|---|---|
| Calculé : | C% 70,99 | H% 8,78 | N% 4,36 |
| Trouvé : | 70,75 | 8,67 | 4,49 |

**Exemple 13 : 1-(3-méthoxy) benzyl 5-octyloxy pyrrolidin 2-one.**

A un mélange de 2,13 g de 5-n-octyloxy pyrrolidin 2-one, 0,62 g de potasse et 0,2 g de bromure de tétrabutylammonium dans 60 cm3 de tétrahydrofuranne, on ajoute entre 20°C et 25°C une solution de 1,57 g de chlorure de 3-méthoxybenzyle dans 15 cm3 de tétrahydrofuranne. On agite pendant 2 heures à température ambiante, filtre, évapore le solvant sous pression réduite. On chromatographie le résidu sur silice (éluant : acétate d'éthyle-n-hexane 1-1) et obtient 2,3 g de produit attendu.

| Analyse : | $C_{20}H_{31}NO_3$ | | |
|---|---|---|---|
| Calculé : | C% 72,03 | H% 9,37 | N% 4,2 |
| Trouvé : | 71,88 | 9,46 | 4,31 |

**Exemple 14 : 1-(4-méthoxy) benzyl 5-n-octyloxy pyrrolidin-2-one.**

A un mélange de 2,13 g de 5-n-octyloxy pyrrolidin-2-one, 0,62 g de potasse et 0,2 g de bromure de tétrabutylammonium dans 60 cm3 de tétrahydrofuranne, on ajoute à 20°C/25°C, une solution de 1,57 g de bromure de 4-méthoxybenzyle dans 15 cm3 de tétrahydrofuranne. On agite 2 heures à température ambiante, filtre et évapore le solvant sous pression réduite. On chromatographie le résidu sur silice (éluant : acétate d'éthyle-n-hexane 1-1) et obtient 2,4 g de produit attendu.

| Analyse : | $C_{20}H_{31}NO_3$ | | |
|---|---|---|---|
| Calculé : | C% 72,03 | H% 9,37 | N% 4,2 |
| Trouvé : | 71,84 | 9,26 | 4,38 |

**Exemple 15 : 1-(3-trifluorométhyl) benzyl 5-n-octyloxy pyrrolidin-2-one.**

A un mélange de 1,65 g de 5-n-octyloxy pyrrolidin-2-one, 0,474 g de potasse et 0,15 g de bromure de tétrabutylammonium dans 45 cm3 de tétrahydrofuranne, on ajoute à 20°C/25°C, une solution de 1,5 g de chlorure de 3-trifluorométhylbenzyle dans 15 cm3 de tétrahydrofuranne. On agite pendant 2 heures, filtre puis évapore à sec sous pression réduite. On chromatographie le résidu sur silice (éluant : acétate d'éthyle-n-hexane 1-1) et obtient 2,3 g de produit attendu.

| Analyse : | $C_{20}H_{28}F_3NO_2$ | | |
|---|---|---|---|
| Calculé : | C% 64,67 | H% 7,60 | N% 3,77 |
| Trouvé : | 64,49 | 7,48 | 3,86 |

**Exemple 16 : 1-(4-trifluorométhyl) benzyl 5-n-octyloxy pyrrolidin-2-one.**

A un mélange de 2,13 g de 5-n-octyloxy pyrrolidin-2-one, 0,2 g de bromure de tétrabutylammonium et 0,62 g de potasse dans 40 cm3 de tétrahydrofuranne, on ajoute sans dépasser 30°C, 2,39 g de bromure de 4-trifluorométhylbenzyle dans 40 cm3 de tétrahydrofuranne. On agite pendant 3 heures à température ambiante, filtre puis évapore le solvant sous pression réduite. On chromatographie le résidu sur silice (éluant : acétate d'éthyle-n-hexane 1-1) et obtient 2,3 g de produit attendu.

| Analyse : | $C_{20}H_{28}F_3NO_2$ | | |
|---|---|---|---|
| Calculé : | C% 64,67 | H% 7,60 | N% 3,77 |
| Trouvé : | 64,45 | 7,49 | 3,89 |

**Exemple 17 : 1-(3-nitro) benzyl 5-n-octyloxy pyrrolidin-2-one.**

A un mélange de 2,2 g de 5-n-octyloxy pyrrolidin-2-one, 0,632 g de potasse et 0,2 g de bromure de tétrabutylammonium dans 60 cm3 de tétrahydrofuranne, on ajoute à 20°C/25°C, 2,2 g de bromure de 3-nitrobenzyle en solution dans 22 cm3 de tétrahydrofuranne. On agite pendant 2 heures à température ambiante, filtre puis évapore à sec sous pression réduite. On chromatographie le résidu sur silice (éluant : acétate d'éthyle-n-hexane 1-1) et obtient 1,6 g de produit attendu.

| Analyse : | $C_{19}H_{29}N_2O_4$ | | |
|---|---|---|---|
| Calculé : | C% 65,49 | H% 8,1 | N% 8,04 |
| Trouvé : | 64,93 | 7,94 | 7,87 |

**Exemple 18 : 1-(4-nitro) benzyl 5-n-octyloxy pyrrolidin-2-one.**

A un mélange de 0,55 g de 5-n-octyloxy pyrrolidin-2-one, 0,05 g de bromure de tétrabutylammonium et 0,16 g de potasse dans 15 cm3 de tétrahydrofuranne, on ajoute sans dépasser 30°C une solution de 0,56 g de bromure de 4-nitrobenzyle dans 10 cm3 de tétrahydrofuranne. On agite pendant 30 minutes, filtre et évapore le solvant sous pression réduite. On chromatographie le résidu sur silice (éluant : acétate d'éthyle-n-hexane 1-1), et obtient 0,2 g de produit attendu.

| Analyse : | $C_{19}H_{28}N_2O_4$ | | |
|---|---|---|---|
| Calculé : | C% 65,49 | H% 8,10 | N% 8,04 |
| Trouvé : | 65,16 | 7,92 | 7,81 |

**Exemple 19 : 1-(4-phényl) benzyl 5-n-octyloxy pyrrolidin-2-one.**

A un mélange de 1,89 g de 5-n-octyloxy pyrrolidin-2-one, 0,55 g de potasse et 0,19 g de bromure de tétrabutylammonium dans 54 cm3 de tétrahydrofuranne, on ajoute entre 20°C et 25°C, une solution de 1,8 g de 4-chlorométhylbiphényle dans 18 cm3 de tétrahydrofuranne. On agite 1 heure à température ambiante, filtre puis évapore le solvant à sec. On chromatographie le résidu sur silice (éluant : acétate d'éthyle-n-hexane 1-1) et obtient 1,8 g de produit attendu.

| | | | | |
|---|---|---|---|---|
| Analyse | : | $C_{25}H_{33}NO_2$ | | |
| Calculé | : | C% 79,11 | H% 8,76 | N% 3,69 |
| Trouvé | : | 78,88 | 7,74 | 3,77 |

**Préparation 1 : 5-isopropyloxy pyrrolidin-2-one.**

On refroidit à −10°C 28,64 g de succinimide dans 1200 cm3 d'isopropanol, on ajoute 32,8 g d'hydrure de bore et de sodium, maintient 4 heures à 0°/−10°C et ajoute à 0°C, +2°C en régulant le pH à 2-3, une solution d'acide chlorhydrique 2N dans l'isopropanol. On agite 2 heures à 0°C, ajoute jusqu'à neutralité une solution d'hydroxyde de potassium dans l'isopropanol. On évapore le solvant sous pression réduite, reprend avec du chloroforme, concentre à sec sous pression réduite et obtient 20,5 g de produit attendu. F = 68-71°C.

**Préparation 2 : 5-n-propyloxy pyrrolidin-2-one.**

On opère comme pour la préparation 1 en utilisant du n-propanol avec 16 g de borohydrure de sodium à une température comprise entre 0°C et −7°C. On obtient 27,5 g de produit attendu. F = 52-54°C.

**Préparation 3 : 5-n-butoxy pyrrolidin-2-one.**

On opère comme pour la préparation 2 en remplaçant l'isopropanol par le n-butanol et en employant 14,32 g de succinimide dans 600 cm3 de n-butanol et 8 g d'hydrure de bore et de sodium. On obtient 7,5 g de produit attendu. F = 36-38°C. Le produit peut aussi être préparé par alcoylation anodique selon un procédé qui est décrit dans Synthesis 4, 315-317 (1980).

**Préparation 4 : 5-pentyloxy pyrrolidin-2-one.**

On chauffe à 65°C pendant 3 heures 2,5 g de 5-hydroxy pyrrolidin-2-one et 1,25 g d'Amberlite IR 120 H® dans 55 cm3 de 1-pentanol. On filtre la résine, distille sous pression réduite à une température de 22°C/24°C sous 0,5 mbar. On chromatographie le résidu sur silice (éluant : acétate d'éthyle) et obtient 2,68 g de produit attendu. F = 42-43°C.

| | | | | |
|---|---|---|---|---|
| Analyse | : | $C_9H_{17}NO_2$ | | |
| Calculé | : | C% 63,13 | H% 10,01 | N% 8,18 |
| Trouvé | : | 63,31 | 9,95 | 8,27 |

**Préparation 5 : 5-hexyloxy pyrrolidin-2-one.**

On chauffe 3 heures à 60°C un mélange de 0,4 g de 5-hydroxy pyrrolidin-2-one, 10 cm3 de n-hexanol et 0,2 g d'Amberlite IR 120 H®, distille le solvant à 25°C sous 0,3 mbar. On chromatographie le résidu sur silice (éluant : acétate d'éthyle), cristallise 0,5 g du résidu dans l'hexane et obtient le produit attendu. F = 35-37°C.

| | | | | |
|---|---|---|---|---|
| Analyse | : | $C_{10}H_{19}NO_2$ | | |
| Calculé | : | C% 64,83 | H% 10,34 | N% 7,56 |
| Trouvé | : | 64,67 | 10,25 | 7,49 |

**Préparation 6 : 5-n-heptyloxy pyrrolidin-2-one.**

On chauffe pendant 4 heures à 60°C un mélange de 8 g de 5-hydroxy pyrrolidin-2-one et 100 cm3 de n-heptanol et 4 g d'Amberlite IR 120 H®, filtre, élimine le solvant sous pression réduite à 45°C/60°C sous 0,3 mbar. On obtient 11,9 g de produit attendu. F = 52-54°C cristallisé de l'hexane.

| | | | |
|---|---|---|---|
| Analyse : | $C_{11}H_{21}NO_2$ | | |
| Calculé : | C% 66,29 | H% 10,62 | N% 7,03 |
| Trouvé : | 66,13 | 10,51 | 6,98 |

**Préparation 7 : 5-n-octyloxy pyrrolidin-2-one.**

On chauffe à 60°C pendant 4 heures 7,5 g de 5-hydroxy pyrrolidin-2-one, 10 cm3 de n-octanol et 4 g d'Amberlite IR 120 H®. On filtre puis distille sous pression réduite pour éliminer le n-octanol. On obtient 5,5 g de produit attendu. F = 36-38°C cristallisé de l'hexane.

| | | | |
|---|---|---|---|
| Analyse : | $C_{12}H_{23}NO_2$ | | |
| Calculé : | C% 67,56 | H% 10,87 | N% 6,57 |
| Trouvé : | 67,32 | 10,73 | 6,69 |

**Préparation 8 : 5-n-nonyloxy pyrrolidin-2-one.**

On agite à 60°C pendant 3 heures un mélange de 5 g de 5-hydroxy pyrrolidin-2-one et 2,5 g de résine Amberlite IR 120 H® dans 100 cm3 de n-nonanol. On refroidit, filtre la résine et distille le solvant à 70°C sous 0,5 mbar. On reprend le résidu dans 50 cm3 de n-hexane que l'on maintient 16 heures à 5°C. On essore et obtient 6,8 g de produit attendu. F = 46-48°C. On le recristallise dans le n-hexane. F = 51-52°C.

| | | | |
|---|---|---|---|
| Analyse : | $C_{13}H_{25}NO_2$ | | |
| Calculé : | C% 68,68 | H% 11,08 | N% 6,16 |
| Trouvé : | 68,33 | 11,20 | 6,30 |

**Préparation 9 : 5-n-décyloxy pyrrolidin-2-one.**

On agite à 60°C pendant 3 heures 3 g de 5-hydroxy pyrrolidin-2-one et 1,5 g de résine Amberlite IR 120 H® dans 60 cm3 de n-décanol. On filtre la résine et distille le solvant à 60°C sous 0,4 mm de Hg. On reprend le résidu dans 50 cm3 de n-hexane, garde 24 heures à −5°C/−10°C, essore et obtient 3,5 g de produit attendu. F = 44-46°C. On le recristallise dans le n-hexane. F = 48-49°C.

| | | | |
|---|---|---|---|
| Analyse : | $C_{14}H_{27}NO_2$ | | |
| Calculé : | C% 69,66 | H% 11,27 | N% 5,80 |
| Trouvé : | 69,39 | 11,30 | 5,84 |

Exemples de compositions pharmaceutiques.

a) On a préparé des comprimés répondant à la formule suivante :

- Produit de l'exemple 6 .......................................... 100 mg
- Excipient q.s. pour un comprimé terminé à .................... 300 mg

(Détail de l'excipient : lactose, amidon de blé, amidon traité, amidon de riz, stéarate de magnésium, talc).

b) On a préparé des gélules répondant à la formule suivante :

- Produit de l'exemple 7 .......................................... 200 mg
- Excipient q.s. pour une gélule terminée à ...................... 300 mg

(Détail de l'excipient : talc, stéarate de magnésium, aérosil®).

ETUDE PHARMACEUTIQUE

Toxicité aiguë et comportement des produits de l'invention.

nous avons utilisé des souris mâles ($CD_1$ Charles Rivers) d'un poids de 22-23 g à jeun depuis 16 heures. Les produits leur furent administrés par voie orale aux doses de 1000-500-250 mg/kg.

L'effet des produits sur le comportement des animaux fut évalué suivant la méthode décrite par Irvin (Psychopharmacologia (1968), 13, 222-257) durant les 8 premières heures et à la 24ème heure.

La mortalité fut relevée pendant les 7 jours suivant le traitement.

La $DL_{50}$ a ainsi été trouvée supérieure à 1000 mg/kg pour les produits des exemples 1, 3 à 7, 9 et 10.

Apprentissage et mémorisation.

Nous avons utilisé des souris mâles ($CD_1$ Charles Rivers) d'un poids de 25-30 g. Les animaux sont placés dans la partie lumineuse d'un box à deux compartiments communicants par une ouverture (G. Galliani, R. Cesana and F. Barzaghi, Med. Sci. Res. 15, 313-314 (1987)).

A l'instant où la souris passe du compartiment lumineux au compartiment obscur, l'ouverture se ferme et elle est immédiatement punie par une décharge électrique aux pattes. L'animal soumis à cette procédure apprend à mémoriser la punition. En fait, si on le remet dans le compartiment lumineux, il évitera ainsi de franchir l'ouverture et de rentrer dans le compartiment obscur.

Pour induire une amnésie rétrograde, les animaux sont soumis immédiatement après l'apprentissage à un électrochoc. Après l'électrochoc, les produits sont administrés par voie orale aux doses de 25 ; 50 ; 100 ; 200 et 400 mg/kg.

Nous avons utilisé de 10 à 50 animaux par dose.

L'effet antiamnésique des produits est évalué 3 heures après le traitement, en utilisant le même protocole que celui utilisé pour l'acquisition.

Le temps mis par l'animal pour retourner dans la chambre obscure (temps limite 180 secondes) est utilisé comme paramètre d'évaluation.

Dans les mêmes conditions expérimentales, les animaux témoins entrent avec un laps de temps de 40-50 secondes.

Les produits actifs sont ceux qui provoquent une augmentation significative du temps de latence.

Les résultats sont exprimés en pourcentages d'augmentation du temps de latence par rapport aux témoins correspondants. Des résultats obtenus avec 2 produits de référence sont fournis.

Les résultats sont les suivants :

Pourcentage d'augmentation du temps de latence par rapport aux témoins

| Produit de l'exemple | Dose mg/kg os | | | | |
|---|---|---|---|---|---|
| | 400 | 200 | 100 | 50 | 25 |
| 6 | 24 | 99* | 56* | 41 | 16 |
| 7 | - | 68* | 94* | 56 | 33 |
| PIRACETAM | - | 20 | 48* | 10 | 19 |
| ANIRACETAM | - | 32 | 88* | 77 | 39 |

* Valeurs statistiquement différentes par rapport aux témoins.

**Conclusion :**

Les produits des exemples 6 et 7 se sont révélés plus actifs que les témoins. Ils améliorent notablement le comportement des animaux dans une gamme de doses plus large que dans le cas de l'Aniracetam ou du Piracetam.

## Revendications

**Revendications pour les Etats contractants suivants : AT, BE, CH/LI, DE, FR, GB, IT, LU, NL, SE**

1. Les composés de formule (I) :

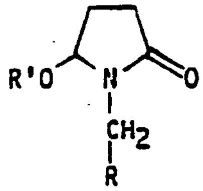

(I)

dans laquelle R' représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b]-pyranyle, benzoxazolyle ou morpholinyle, R étant éventuellement substitué par un ou plusieurs substituants choisis dans le groupe constitué par le radical hydroxy, le radical acétoxy, le radical méthoxy ou le radical benzyloxy, le radical méthyle, éthyle, propyle ou isopropyle, le radical éthényle ou le radical éthynyle, le fluor, le chlore ou le brome, les groupements $CF_3$, $SCF_3$, $OCF_3$, $NO_2$, $NH_2$ ou $C{\equiv}N$, le

radical phényle, les groupements acyles ou alcoxycarbonyles renfermant de 2 à 8 atomes de carbone et les groupements alcoylsulfonyles renfermant de 1 à 6 atomes de carbone, à la condition que si R est un radical phényle non substitué, R' ne représente ni un atome d'hydrogène, ni un radical méthyle, ni un radical éthyle.

2. Les composés de formule (I) tels que définis à la revendication 1, dans lesquels R représente un radical phényle éventuellement substitué.

3. Les composés de formule (I) tels que définis à la revendication 1 ou 2, dans lesquels R' représente un radical alcoyle linéaire, ramifié ou cyclique, renfermant de 3 à 12 atomes de carbone.

4. Les composés de formule (I) tels que définis à la revendication 3, dans lesquels R' représente un radical n-pentyle, n-hexyle, n-heptyle ou n-octyle.

5. Les composés définis à la revendication 1 dont les noms suivent :

— la 1-benzyl 2-oxo 5-n-heptyloxy pyrrolidine,

— la 1-benzyl 2-oxo 5-n-octyloxy pyrrolidine.

6. A titre de médicaments, les composés répondant à la formule générale (I) de la revendication 1.

7. A titre de médicaments, les composés définis à la revendication 5.

8. Les compositions pharmaceutiques renfermant comme principe actif au moins un médicament défini à la revendication 6 ou 7.

9. Procédé de préparation des composés de formule (I) tels que définis à l'une quelconque des revendications 1 à 5, caractérisé en ce que l'on soumet un composé de formule (II) :

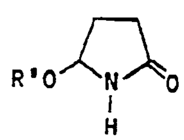

(II)

dans laquelle R' conserve la même signification que précédemment à l'action d'un composé de formule III :

$RCH_2Hal$ (III)

dans laquelle R conserve la même signification que précédemment et Hal représente un atome d'halogène, en présence d'une base pour obtenir le composé de formule (I) correspondant.

**Revendications pour les Etats contractants suivants : ES, GR**

1. Procédé de préparation des composés de formule (I) :

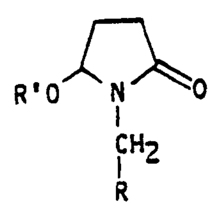

(I)

dans laquelle R' représente un atome d'hydrogène, un radical alcoyle, linéaire, ramifié ou cyclique renfermant jusqu'à 12 atomes de carbone, un radical alcényle renfermant de 2 à 8 atomes de carbone, un radical acyle renfermant de 1 à 6 atomes de carbone ou un radical benzyle ou phénéthyle et R représente un radical phényle ou biphénylyle ou un radical furyle, thiényle, pyranyle, pyridyle, benzofuranyle, isobenzofuranyle, chromanyle, isochromanyle, chroményle, xanthényle, phénoxathiényle, oxazolyle, isoxazolyle, furazanyle, phénoxazinyle, thiéno [2,3-b] furanyle, 2H-furo [3,2-b]-pyranyle, benzoxazolyle ou morpholinyle, R étant éventuellement substitué par un ou plusieurs substituants choisis dans le groupe constitué par le radical hydroxy, le radical acétoxy, le radical méthoxy ou le radical benzyloxy, le radical méthyle, éthyle, propyle ou isopropyle, le radical éthényle ou le radical éthynyle, le fluor, le chlore ou le brome, les groupements $CF_3$, $SCF_3$, $OCF_3$, $NO_2$, $NH_2$ ou $C\equiv N$, le radical phényle, les groupements acyles ou alcoxycarbonyles renfermant de 2 à 8 atomes de carbone et les groupements alcoylsulfonyles renfermant de 1 à 6 atomes de carbone, à la condition que si R est un radical phényle non substitué, R' ne représente ni un atome d'hydrogène, ni un radical méthyle, ni un radical éthyle, caractérisé en ce que l'on soumet un composé de formule (II) :

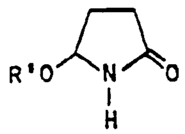

(II)

dans laquelle R′ conserve la même signification que précédemment, à l'action d'un composé de formule III :

$$RCH_2Hal \quad (III)$$

dans laquelle R conserve la même signification que précédemment et Hal représente un atome d'halogène, en présence d'une base pour obtenir le composé de formule (I) correspondant.

2. Procédé selon la revendication 1, caractérisé en ce que l'on utilise au départ un composé de formule (III) dans laquelle R représente un radical phényle éventuellement substitué.

3. Procédé selon la revendication 1 ou 2, caractérisé en ce que l'on utilise au départ un composé de formule (II) dans laquelle R′ représente un radical alcoyle linéaire, ramifié ou cyclique, renfermant de 3 à 12 atomes de carbone.

4. Procédé selon la revendication 3, caractérisé en ce que l'on utilise au départ un composé de formule (II) dans laquelle R′ représente un radical n-pentyle, n-hexyle, n-heptyle ou n-octyle.

5. Procédé selon l'une quelconque des revendications 1 à 4, caractérisé en ce que l'on utilise au départ un composé de formule (II) dans laquelle R′ représente un radical n-heptyle ou n-octyle et un composé de formule (III) dans laquelle R représente un radical phényle.

## Patentansprüche

### Patentansprüche für folgende Vertragsstaaten : AT, BE, CH, DE, FR, GB, IT, LI, LU, NL, SE

1. Verbindungen der Formel (I)

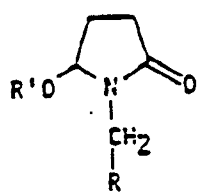

(I)

worin R′ ein Wasserstoffatom, einen linearen, verzweigten oder cyclischen Alkylrest mit bis zu 12 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest bedeutet und R einen Phenyl- oder Biphenylylrest oder einen Furyl-, Thienyl-, Pyranyl-, Pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno-[2,3-b]-furanyl-, 2H-Furo-[3,2-b]-pyranyl-, Benzoxazolyl- oder Morpholinylrest wiedergibt, wobei R gegebenenfalls durch einen oder mehrere Substituenten substituiert ist, ausgewählt unter der Hydroxygruppe, der Acetoxygruppe, der Methoxygruppe oder dem Benzyloxyrest, dem Methyl-, Ethyl-, Propyl- oder Isopropylrest, dem Ethenylrest oder dem Ethinylrest, dem Fluor, dem Chlor oder dem Brom, den Gruppen $CF_3$, $SCF_3$, $OCF_3$, $NO_2$, $NH_2$ oder C≡N, der Phenylgruppe, den Acyl- oder Alkoxycarbonylgruppen mit 2 bis 8 Kohlenstoffatomen und den Alkylsulfonylgruppen mit 1 bis 6 Kohlenstoffatomen, mit der Maßgabe, daß, wenn R eine nicht substituierte Phenylgruppe bedeutet, R′ weder ein Wasserstoffatom noch einen Methylrest noch einen Ethylrest bedeuten kann.

2. Verbindungen der Formel (I) gemäß Anspruch 1, worin R für einen gegebenenfalls substituierten Phenylrest steht.

3. Verbindungen der Formel (I) gemäß Anspruch 1 oder 2, worin R′ einen linearen, verzweigten oder cyclischen Alkylrest mit 3 bis 12 Kohlenstoffatomen bedeutet.

4. Verbindungen der Formel (I) gemäß Anspruch 3, worin R′ einen n-Pentyl-, n-Hexyl-, n-Heptyl- oder n-Octylrest bedeutet.

5. Verbindungen gemäß Anspruch 1 mit den folgenden Bezeichmungen :

1-Benzyl-2-oxo-5-n-heptyloxypyrrolidin,

1-Benzyl-2-oxo-5-n-octyloxypyrrolidin.

6. Als Arzneimittel die Verbindungen der allgemeinen Formel (I) gemäß Anspruch 1.

7. Als Arzneimittel die Verbindungen gemäß Anspruch 5.

8. Pharmazeutische Zusammensetzungen,enthaltend als Wirkstoff zumindest ein Arzneimittel gemäß Anspruch 6 oder 7.

9. Verfahren zur Herstellung der Verbindungen der Formel (I) gemäß einem der Ansprüche 1 bis 5, dadurch gekennzeichnet, daß man eine Verbindung der Formel (II)

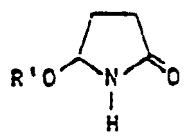

(II)

worin R' die vorstehend angegebene Bedeutung besitzt, der Einwirkung einer Verbindung der Formel (III)

$RCH_2Hal$ (III)

worin R die vorstehend angegebene Bedeutung besitzt und Hal für ein Halogenatom steht, in Anwesenheit einer Base unterzieht, um zu der entsprechenden Verbindung der Formel (I) zu gelangen.

**Patentansprüche für folgende Vertragsstaaten : ES, GR**

1. Verfahren zur Herstellung der Verbindungen der Formel (I)

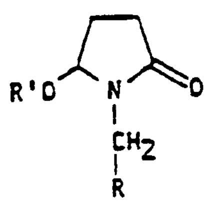

(I)

worin R' ein Wasserstoffatom, einen linearen, verzweigten oder cyclischen Alkylrest mit bis zu 12 Kohlenstoffatomen, einen Alkenylrest mit 2 bis 8 Kohlenstoffatomen, einen Acylrest mit 1 bis 6 Kohlenstoffatomen oder einen Benzyl- oder Phenethylrest bedeutet und R einen Phenyl- oder Biphenylylrest oder einen Furyl-, Thienyl-, pyranyl-, pyridyl-, Benzofuranyl-, Isobenzofuranyl-, Chromanyl-, Isochromanyl-, Chromenyl-, Xanthenyl-, Phenoxathienyl-, Oxazolyl-, Isoxazolyl-, Furazanyl-, Phenoxazinyl-, Thieno-[2,3-b]-furanyl-, 2H-Furo-[3,2-b]-pyranyl-, Benzoxazolyl- oder Morpholinylrest wiedergibt, wobei R gegebenenfalls durch einen oder mehrere Substituenten substituiert ist, ausgewählt unter der Hydroxygruppe, der Acetoxygruppe, der Methoxygruppe oder der Benzyloxygruppe, dem Methyl-, Ethyl-, Propyl- oder Isopropylrest, dem Ethenylrest oder dem Ethinylrest, dem Fluor, dem Chlor oder dem Brom, den Gruppen $CF_3$, $SCF_3$, $OCF_3$, $NO_2$, $NH_2$ oder C≡N, dem Phenylrest, den Acyl- oder Alkoxycarbonylresten mit 2 bis 8 Kohlenstoffatomen und den Alkylsulfonylgruppen mit 1 bis 6 Kohlenstoffatomen, mit der Maßgabe, daß, wenn R eine nicht substituierte Phenylgruppe bedeutet, R' weder ein Wasserstoffatom noch einen Methylrest noch einen Ethylrest bedeuten kann, dadurch gekennzeichnet, daß man eine Verbindung der Formel (II)

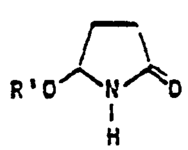

(II)

worin R' die vorstehend angegebene Bedeutung besitzt, der Einwirkung einer Verbindung der Formel (III)

$RCH_2Hal$ (III)

worin R die vorstehend angegebene Bedeutung besitzt und Hal für ein Halogenatom steht, in Anwesenheit einer Base unterzieht, um zu der entsprechenden Verbindung der Formel (I) zu gelangen.

2. Verfahren gemäß Anspruch 1, dadurch gekennzeichnet, daß man von einer Verbindung der Formel (III) ausgeht, worin R für einen gegebenenfalls substituierten Phenylrest steht.

3. Verfahren gemäß Anspruch 1 oder 2, dadurch gekennzeichnet, daß man von einer Verbindung der Formel (II) ausgeht, worin R' für einen linearen, verzweigten oder cyclischen Alkylrest mit 3 bis 12 Kohlenstoffatomen steht.

4. Verfahren gemäß Anspruch 3, dadurch gekennzeichnet, daß man von einer Verbindung der Formel (II) ausgeht, worin R' für einen n-Pentyl-, n-Hexyl-, n-Heptyl- oder n-Octylrest steht.

5. Verfahren gemäß einem der Ansprüche 1 bis 4, dadurch gekennzeichnet, daß man von einer Verbindung der Formel (II), worin R' für einen n-Heptyl- oder n-Octylrest steht, und einer Verbindung der Formel (III), worin R für einen Phenylrest steht, ausgeht.

## Claims

### Claims for the following Contracting States : AT, BE, CH, DE, FR, GB, IT, LI, LU, NL, SE

1. The compounds of formula (I) :

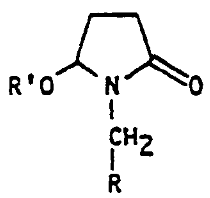

(I)

in which R' represents a hydrogen atom, a linear, branched or cyclic alkyl radical containing up to 12 carbon atoms, an alkenyl radical containing 2 to 8 carbon atoms, an acyl radical containing 1 to 6 carbon atoms, or a benzyl or phenethyl radical and R represents a phenyl or biphenylyl radical or a furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl radical, R being optionally substituted by one or more substituents chosen from the group constituted by hydroxy, acetoxy, methoxy or benzyloxy radicals, methyl, ethyl, propyl or isopropyl radicals, ethenyl or ethynyl radicals, fluorine, chlorine or bromine, $CF_3$, $SCF_3$, $OCF_3$, $NO_2$, $NH_2$ or C≡N groups, phenyl radical, acyl or alkoxycarbonyl groups containing 2 to 8 carbon atoms and alkylsulphonyl groups containing 1 to 6 carbon atoms, on the condition that if R is a non-substituted phenyl radical, R' cannot represent either a hydrogen atom, or a methyl radical, or an ethyl radical.

2. The compounds of formula (I) as defined in claim 1, in which R represents an optionally substituted phenyl radical.

3. The compounds of formula (I) as defined in claim 1 or 2, in which R' represents a linear, branched or cyclic alkyl radical containing 3 to 12 carbon atoms.

4. The compounds of formula (I) as defined in claim 3, in which R' represents an n-pentyl, n-hexyl, n-heptyl or n-octyl radical.

5. The compounds defined in claim 1 the names of which follow :

— 1-benzyl-2-oxo-5-n-heptyloxy pyrrolidine,

— 1-benzyl-2-oxo-5-n-octyloxy pyrrolidine.

6. As medicaments, the compounds corresponding to general formula (I) of claim 1.

7. As medicaments, the compounds defined in claim 5.

8. The pharmaceutical compositions containing as active ingredient at least one medicament defined in claim 6 or 7.

9. Preparation process for the compounds of formula (I) as defined in any one of claims 1 to 5, characterized in that a compound of formula (II) :

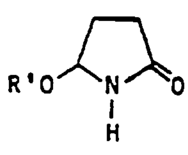

(II)

in which R′ retains the same meaning as previously, is subjected to the action of a compound of formula (III) :

$RCH_2Hal$ (III)

in which R retains the same meaning as previously and Hal represents a halogen atom, in the presence of a base, in order to obtain the corresponding compound of formula (I).

**Claims for the following Contracting States : ES, GR**

1. Preparation process for compounds of formula (I) :

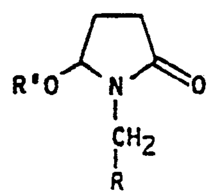

(I)

in which R′ represents a hydrogen atom, a linear, branched or cyclic radical containing up to 12 carbon atoms, an alkenyl radical containing 2 to 8 carbon atoms, an acyl radical containing 1 to 6 carbon atoms, or a benzyl or phenethyl radical and R represents a phenyl or biphenylyl radical or a furyl, thienyl, pyranyl, pyridyl, benzofuranyl, isobenzofuranyl, chromanyl, isochromanyl, chromenyl, xanthenyl, phenoxathienyl, oxazolyl, isoxazolyl, furazanyl, phenoxazinyl, thieno-[2,3-b]-furanyl, 2H-furo-[3,2-b]-pyranyl, benzoxazolyl or morpholinyl radical, R being optionally substituted by one or more substituents chosen from the group constituted by hydroxy, acetoxy, methoxy or benzyloxy radicals, methyl, ethyl, propyl or isopropyl radicals, ethenyl or ethynyl radicals, fluorine, chlorine or bromine, $CF_3$, $SCF_3$, $OCF_3$, $NO_2$, $NH_2$ or C≡N groups, phenyl radical, acyl or alkoxycarbonyl groups containing 2 to 8 carbon atoms and alkylsulphonyl groups containing 1 to 6 carbon atoms, on the condition that if R is a non-substituted phenyl radical, R′ cannot represent either a hydrogen atom, or a methyl radical, or an ethyl radical, characterized in that a compound of formula (II) :

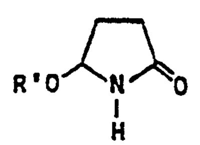

(II)

in which R′ retains the same meaning as previously, is subjected to the action of a compound of formula (III) :

$RCH_2Hal$ (III)

in which R retains the same meaning as previously and Hal represents a halogen atom, in the presence of a base in order to obtain the corresponding compound of formula (I).

2. Process according to claim 1, characterized in that a compound of formula (III) in which R represents an optionally substituted phenyl radical is used at the start.

3. Process according to claim 1 or 2, characterized in that a compound of formula (II) in which R′ represents a linear, branched or cyclic alkyl radical containing 3 to 12 carbon atoms is used at the start.

4. Process according to claim 3, characterized in that a compound of formula (II) in which R′ represents an n-pentyl, n-hexyl, n-heptyl or n-octyl radical is used at the start.

5. Process according to any one of claims 1 to 4, characterized in that a compound of formula (II) in which R′ represents an n-heptyl or n-octyl radical and a compound of formula (III) in which R represents a phenyl radical are used at the start.